# EUROPÄISCHE PATENTSCHRIFT

(11) **EP 3 150 190 B1**
(45) Veröffentlichungstag und Bekanntmachung des Hinweises auf die Patenterteilung: **12.02.2020**
(21) Anmeldenummer: 16189116.3
(22) Anmeldetag: 16.09.2016
(51) Int. Cl.: A61K 8/49, A61Q 17/04, A61K 8/35

(54) **SONNENSCHUTZMITTEL MIT REDUZIERTER TEXTILVERFLECKUNG DURCH 4-(TERT.-BUTYL)-4´-METHOXY-DI-BEN-ZOYLMETHAN**
SUNSCREEN WITH REDUCED TEXTILE STAINING DUE TO 4- (TERT-BUTYL) -4'-METHOXY-DI-BEN-ZOYLMETHANE
PROTECTION SOLAIRE COMPRENANT UN AGENT DE RÉDUCTION DE TACHES PAR 4-(TERT.-BUTYLE)-4-METHYLDIBENZOYLMETHANE

(30) Priorität: 01.10.2015 DE 102015219008
(43) Veröffentlichungstag der Anmeldung: 05.04.2017
(73) Patentinhaber: Beiersdorf AG, 20253 Hamburg (DE)
(72) Erfinder: Weinert, Katrin, 22763 Hamburg (DE); Borchers, Kathrin, 21614 Buxtehude (DE); Bleckmann, Andreas, 22926 Ahrensburg (DE)

(56) Entgegenhaltungen:
- EP-A1- 3 269 425
- WO-A1-2012/078961
- DE-A1-102013 209 904
- US-A1- 2011 067 188
- Clariant: "Antidandruff Active Ingredient Octopirox", Salzbach, 11. Juni 2015 (2015-06-11), Seiten 1-16, XP055319009, Gefunden im Internet: URL:http://www.myskinrecipes.com/shop/atta chment.php?id_attachment=217 [gefunden am 2016-11-14]

## Beschreibung

Die vorliegende Erfindung betrifft ein Verfahren zur Erleichterung der Auswaschbarkeit von 4-(tert.-Butyl)-4'-methoxydibenzoylmethan enthaltenden kosmetischen Zubereitungen aus Textilien, dadurch gekennzeichnet, dass dem Kosmetikum 1-Hydroxy-4-methyl-6-(2,4,4-trimethylpentyl)-2(1 H)-pyridon (Pirocton) oder dessen Monoethanolamin-Salz zugesetzt wird sowie die Verwendung von 1-Hydroxy-4-methyl-6-(2,4,4-trimethylpentyl)-2(1 H)-pyridon (Pirocton) oder dessen Monoethanolamin-Salz in 4-(tert.-Butyl)-4'-methoxydibenzoylmethan enthaltenden kosmetischen Zubereitungen zur Erleichterung der Auswaschbarkeit der UV-Lichtschutzfilter aus mit den Zubereitungen kontaminierten Textilien.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurde daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Kosmetische Zubereitungen wie Sonnenschutzzubereitungen, die auf die Haut aufgetragen werden, kommen regelmäßig (beabsichtigt oder unbeabsichtigt) mit Kleidungsstücken und Wäschestücken (z.B. Handtücher) in Kontakt, an denen sie (z.B. als "Abrieb" oder weil sie von den Faserstoffen "aufgesaugt" werden) zum Teil haften bleiben. Auf diese Weise entstehen, je nach Art der Inhaltsstoffe, insbesondere auf hellen Textilien Flecken und Verfärbungen. Diese Verfärbungen werden insbesondere durch nicht-wasserlösliche UVA- und Breitbandfilter wie 4-(tert.-Butyl)-4'-methoxydibenzoylmethan hervorgerufen. Die Verfleckungen sind durch Waschen mit herkömmlichen Waschmitteln kaum zu entfernen und verstärken sich während des Waschprozesses durch Wechselwirkungen mit Ionen des Waschwassers sogar noch.

Es war daher die Aufgabe der vorliegenden Erfindung, die Nachteile des Standes der Technik zu beseitigen und ein Verfahren bzw. eine Verwendung für eine kosmetische Zubereitung (insbesondere ein Sonnenschutzmittel) enthaltend nicht-wasserlösliche UV-A Filter wie 4-(tert.-Butyl)-4'-methoxydibenzoylmethan zu entwickeln, damit diese UV-Filter sich leichter aus den mit der Zubereitung kontaminierten Textilen herauswaschen lassen.

Überraschend gelöst wird die Aufgabe durch ein Verfahren zur Erleichterung der Auswaschbarkeit von 4-(tert.-Butyl)-4'-methoxydibenzoylmethan enthaltenden kosmetischen Zubereitungen aus Textilien, dadurch gekennzeichnet, dass dem Kosmetikum 1-Hydroxy-4-methyl-6-(2,4,4-trimethylpentyl)-2(1H)-pyridon (Pirocton) und/oder dessen Monoethanolamin-Salz zugesetzt wird.

Überraschend wird die Aufgabe ferner gelöst durch die Verwendung von 1-Hydroxy-4-methyl-6-(2,4,4-trimethylpentyl)-2(1H)-pyridon (Pirocton) und/oder dessen Monoethanolamin-Salz in 4-(tert.-Butyl)-4'-methoxydibenzoylmethan enthaltenden kosmetischen Zubereitungen zur Erleichterung der Auswaschbarkeit der UV-Lichtschutzfilter aus mit den Zubereitungen kontaminierten Textilien.

Zwar kennt der Stand der Technik die DE102004011111, doch konnte diese Schrift nicht den Weg zur vorliegenden Erfindung weisen, weil sie sich mit dem Effekt der Fotostabilisierung von Dibenzoylmethanderivaten befasst. Darüber hinaus kennt der Fachmann die WO2012078961, 102010054918, DE 10201005465, DE 102010054866, DE 19639817, EP0928193, EP 1088546 und EP0463780, die ebenfalls nicht den Weg zur vorliegenden Erfindung weisen konnten.

Im Rahmen der vorliegenden Offenbarung beziehen sich die Begriffe "erfindungsgemäß", "erfindungsgemäß vorteilhaft", "erfindungsgemäße Zubereitung" etc. immer auf das erfindungsgemäße Verfahren und auf die erfindungsgemäße Verwendung. "Erfindungsgemäße Zubereitungen" etc. sind somit Zubereitungen, in denen das erfindungsgemäße Verfahren oder die erfindungsgemäße Verwendung verwirklicht wird. Diese Formulierung bezieht sich damit immer auf das erfindungsgemäße Verfahren bzw. auf die erfindungsgemäße Verwendung.

Es ist erfindungsgemäß vorteilhaft, wenn die Zubereitung 4-(tert.-Butyl)-4'-methoxydiben-zoylmethan in einer Menge von 0,01 bis 5 Gewichts-%, bezogen auf das Gesamtgewicht der Zubereitung, enthält. Erfindungsgemäß bevorzugt ist dabei ein Gehalt von 2 bis 5 Gewichts-%, bezogen auf das Gesamtgewicht der Zubereitung.

Darüber hinaus ist es vorteilhaft im Sinne der vorliegenden Erfindung, wenn die Zubereitung 1-Hydroxy-4-methyl-6-(2,4,4-trimethylpentyl)-2(1 H)-pyridon (Pirocton) in einer Gesamtmenge von 0,01 bis 0,5 Gewichts-%, bezogen auf das Gesamtgewicht der Zubereitung, enthält. Erfindungsgemäß bevorzugt ist dabei ein Gehalt von 0,25 bis 0,5 Gewichts-%, bezogen auf das Gesamtgewicht der Zubereitung.

Der Zusatz von Pirocton kann dabei erfindungsgemäß auf zwei Wegen erfolgen. Zum einen kann das Piroton per se der Zubereitung zugesetzt werden. Darüber hinaus kann es aber auch in Form seines Monoethanolamin-Salzes zugesetzt werden. Beide Wege sind erfindungsgemäß.

Die erfindungsgemäß bevorzugte Variante ist jedoch dadurch gekennzeichnet, dass das 1-Hydroxy-4-methyl-6-(2,4,4-trimethylpentyl)-2(1 H)-pyridon (Pirocton) in Form seines Monoethanolamin-Salzes (Piroctone Olamine) eingesetzt wird.

Wird das Pirocton in Form von Piroctone Olamine zugefügt, so ist es erfindungsgemäß vorteilhaft, diesen Rohstoff in einer Menge von 0,01 bis 0,5 Gewichts-%, bezogen auf das Gesamtgewicht der Zubereitung und bevorzugt in einer Menge von 0,1 bis 0,5 Gewichts-%, bezogen auf das Gesamtgewicht der Zubereitung, einzusetzen.

Erfindungsgemäß vorteilhaft ist es, wenn das Gewichtsverhältnis von 4-(tert.-Butyl)-4'-methoxy-dibenzoylmethan zu 1-Hydroxy-4-methyl-6-(2,4,4-trimethylpentyl)-2(1 H)-pyridon (Pirocton) von 10:1 bis 100:1 beträgt.

Die erfindungsgemäße Zubereitung kann in Form eines alkoholischen Sprays oder Gels oder in Form einer Emulsion vorliegen.

Liegt die Zubereitung in Form eines alkoholischen Sprays oder Gels vor, so ist es erfindungsgemäß vorteilhaft, wenn der Gehalt an Ethanol von 20 bis 70 Gewichts-%, bezogen auf das Gesamtgewicht der Zubereitung beträgt.

Liegt die erfindungsgemäße Zubereitung in Form einer Emulsion vor, so ist es erfindungsgemäß vorteilhaft, wenn es sich dabei um eine O/W-Emulsion handelt.

Darüber hinaus sind derartige Emulsionen als erfindungsgemäß vorteilhaft dadurch gekennzeichnet, dass die Zubereitung einen oder mehrere Emulgatoren gewählt aus der Gruppe der Verbindungen Glycerylstearatcitrat, Cetearylalkohol, Natriumcetearylsulfat, Glycerylstearat, Cetearylsulfosuccinat, Natriumstearoylglutamat, Polyglyceryl-3-methylglucosedistearat, Sucrose Polystearate, Stearinsäure, Polyglyceryl-10 Stearat, Kaliumcetylphosphat, enthält.

Es ist vorteilhaft im Sinne der vorliegenden Erfindung, wenn die kosmetische Zubereitung dadurch gekennzeichnet ist, dass die Zubereitung einen oder mehrere UV-Filter enthält, die gewählt werden aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)-benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; 2-(4'-Diethylamino-2'-hydoxybenzoyl)-benzoesäurehexylester; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 4,4',4"-(1,3,5-Triazin-2,4,6-triyltriimino)-tris-benzoësäure-tris(2-ethylhe-xylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titandioxid; Zinkoxid.

Erfindungsgemäß bevorzugt ist es dabei, wenn die erfindungsgemäße Zusammensetzung 2-(4'-Diethylamino-2'-hydoxybenzoyl)-benzoesäurehexylester und/oder 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin), enthält.

Die erfindungsgemäß vorteilhafte Einsatzkonzentration für 2-(4'-Diethylamino-2'-hydoxybenzoyl)-benzoesäurehexylester beträgt dabei von 0,1 bis 10 Gewichts-%, bezogen auf das Gesamtgewicht der Zubereitung.

Die erfindungsgemäß bevorzugte Einsatzkonzentration für 2-(4'-Diethylamino-2'-hydoxybenzoyl)-benzoesäurehexylester beträgt dabei von 0,5 bis 5 Gewichts-%, bezogen auf das Gesamtgewicht der Zubereitung.

Es ist erfindungsgemäß vorteilhaft, wenn die Zubereitung eine oder mehrere Verbindungen gewählt aus der Gruppe der Verbindungen Ethylhexylglycerin, Polyglyceryl-2 Caprate, Propylenglycol, Butylenglycol, 2-Methylpropan-1,3-diol, 1,2-Pentandiol, 1,2-Hexandiol, 1,2-Octandiol und/oder 1,2-Decandiol enthält.

Darüber hinaus ist es erfindungsgemäß von Vorteil, wenn die Zubereitung Phenoxyethanol und/oder Methylparaben enthält.

Dabei ist es erfindungsgemäß bevorzugt, wenn die Zubereitung Phenoxyethanol in einer Konzentration von 0,01 bis 1 Gewichts-%, bezogen auf das Gesamtgewicht der Zubereitung, enthält.

Es ist erfindungsgemäß von Vorteil, wenn die Zubereitung einen oder mehrere Wirkstoffe gewählt aus der Gruppe der Verbindungen Magnolienextrakt, alpha-Liponsäure, Folsäure, Phytoen, D-Biotin, Coenzym Q10, alpha-Glucosylrutin, Carnitin, Carnosin, Polydocanol, natürliche und/oder synthetische Isoflavonoide, Flavonoide, Kreatin, Kreatinin, Taurin, β-Alanin, Tocopherylacetat, Panthenol, Magnolol, Honokiol, Harnstoff, Hyaluronsäure, Dihydroxyaceton, 8-Hexadecen-1,16-dicarbonsäure, Glycyrrhetinsäure, Glucosylglyceride und/oder Licochalcon A enthält.

Erfindungsgemäß besonders bevorzugt ist dabei der Einsatz von Glycyrrhetinsäure bzw. Glycyrrhetinsäure enthaltender Pflanzenextrakte (z.B. *Glycyrrhiza glabra*).

Erfindungsgemäß bevorzugt ist es, wenn die Zubereitung frei ist von Propyl- und Butylparaben, 3-Iod-2-propinylbutylcarbamat, 3-(4-Methylbenzyliden)-campher und 2-Hydroxy-4-methoxybenzophenon (Oxybenzon).

Vorteilhaft enthält die erfindungsgemäße Zubereitung Glycerin. Die erfindungsgemäß vorteilhafte Einsatzkonzentration für Glycerin beträgt in den erfindungsgemäßen Zubereitungen von 0,01 bis 15 Gewichts-%, bezogen auf das Gesamtgewicht der Zubereitung.

Erfindungsgemäß vorteilhafte Ausführungsformen erhält man nicht zuletzt dadurch, dass die Zubereitung C12-15 Alkylbenzoat, Cyclomethicon, Octyldodecanol, Capryl/Caprinsäure Triglyceride, Butylenglykol Dicaprylat/Dicaprat, Phenethylbenzoat, Cocoglyceride, Di-n-butyladipat und/oder Diisopropyladipat enthält.

Erfindungsgemäß vorteilhafte Ausführungsformen der vorliegenden Erfindung sind dadurch gekennzeichnet, dass die Zubereitung einen oder mehrere Komplexbildner enthält. Diese können vorteilhaft gewählt werden aus der Gruppe der Verbindungen der EDTA, Aminopolycarboxylate, Polyphosphonate, Polyphosphate und komplexbildenden Säuren.

Dabei ist es erfindungsgemäß bevorzugt, wenn als Komplexbildner eine oder mehrere der Verbindungen aus der Gruppe
- Ethylendiamintetraessigsäure/ EDTA
- 1-Hydroxyethan-(1,1-diphosphonsäure)/ HEDP
- Aminotrimethylenphosphonsäure/ ATMP
- Diethylentriaminpenta(methylenphosphonsäure)/ DTPMP
- Ethylendiamintetra(methylenphosphonsäure/ EDTMP
- Phosphonobutan-tricarbonsäure/ PBTC
- Iminodisuccinat
- Natriumpolyphosphat
- Tetranatriumpyrophosphat
- Hydroxamsäure
- Polygalacturonsäure
- Bernsteinsäure
- Ameisensäure
- Äpfelsäure
   und/oder deren Alkalisalze eingesetzt werden.

Erfindungsgemäß besonders bevorzugte Ausführungsformen der vorliegenden Erfindung sind dadurch gekennzeichnet, dass als Komplexbildner eine oder mehrere der Verbindungen aus der Gruppe
- Ethylendiamintetraessigsäure/ EDTA
- Diethylentriaminpenta(methylenphosphonsäure)/ DTPMP
- Ethylendiamintetra(methylenphosphonsäure/ EDTMP
- Aminotrimethylenphosphonsäure/ ATMP
- Iminodisuccinat
- Natriumpolyphosphat
- Tetranatriumpyrophosphat
- Bernsteinsäure
   und/oder deren Alkalisalze eingesetzt werden.

Als erfindungsgemäß vorteilhafte Alkalisalze gelten dabei Natrium- und Kaliumsalze, wobei die Natriumsalze erfindungsgemäß bevorzugt werden.

Es ist vorteilhaft im Sinne der vorliegenden Erfindung, wenn die erfindungsgemäße Zubereitung einen oder mehrere Komplexbildner in einer Gesamtmenge von 0,1 bis 3 Gewichts-%, bezogen auf das Gesamtgewicht der Zubereitung, enthält.

Es ist bevorzugt im Sinne der vorliegenden Erfindung, wenn die erfindungsgemäße Zubereitung einen oder mehrere Komplexbildner in einer Gesamtmenge von 0,5 bis 1,5 Gewichts-%, bezogen auf das Gesamtgewicht der Zubereitung, enthält.

Darüber hinaus ist es vorteilhaft im Sinne der vorliegenden Erfindung, wenn die Zubereitung eine oder mehrere Verbindungen aus der Gruppe der Polysaccharide enthält.

Es ist in jedem Falle erfindungsgemäß vorteilhaft, wenn die Zubereitung Polysaccharide in einer Gesamtmenge von 0,01 bis 2 Gewichts-%, bezogen auf das Gesamtgewicht der Zubereitung, enthält.

Es ist vorteilhaft im Sinne der vorliegenden Erfindung, wenn die Zubereitung ein oder mehrere Verdickungsmittel enthält.

Erfindungsgemäß bevorzugt werden dabei ein oder mehrere Verdickungsmittel gewählt aus der Gruppe der Verbindungen Ammonium Acryloyldimethyltaurate/VP Copolymer, Ammonium Acryloyldimethyltaurate/ Beheneth-25 Methacrylate Copolymer, Hydroxypropylcellulose Vinylpyrrolidon/Acrylsäure-Copolymer enthält.

Es ist insbesondere von Vorteil, die Filmbildner aus der Gruppe der Polymere auf Basis von Polyvinylpyrrolidon (PVP) 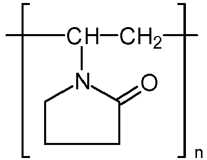 zu wählen. Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind.Erfindungsgemäß besonders bevorzugt ist auch das Vinylpyrrolidon/Triaconten-Copolymer Antaron WP-660.

Ebenfalls vorteilhaft sind weitere polymere Filmbildner, wie beispielsweise Natriumpolystryrensulfonat, welches unter der Handelsbezeichnung Flexan 130 bei der National Starch and Chemical Corp. erhältlich ist, und/oder Polyisobuten, erhältlich bei Rewo unter der Handelsbezeichnung Rewopal PIB1000. Weitere geeignete Polymere sind z.B. Polyacrylamide (Seppigel 305), Polyvinylalkohole, PVP, PVP / VA Copolymere, Polyglycole, Acrylat/Octylacralymid Copolymer (Dermacryl 79). Ebenfalls vorteilhaft ist die Verwendung von Hydriertem Rizinusöl Dimerdilinoleat (CAS 646054-62-8, INCI Hydrogenated Castor Oil Dimer Dilinoleate), das bei der Firma Kokyu Alcohol Kogyo unter dem Namen Risocast DA-H erworben werden kann oder aber auch PPG-3 Benzylethermyristat (CAS 403517-45-3), das unter dem Handelsnamen Crodamol STS bei der Firma Croda Chemicals erworben werden kann.

### Vergleichsversuch

Mit dem folgenden Versuch konnte der erfindungsgemäße Effekt beispielhaft belegt werden: Es wurden jeweils 0,5% des erfindungsgemäßen Hilfsstoffs Pirocton bzw. dessen Monoethanolamin-Salzes zu einer Butyl Methoxydibenzoylmethane-enthaltenden Formulierung zugesetzt und die verfleckungsreduzierende Wirkung (Reduktion db) im Vergleich zu einer Formulierung ohne den erfindungsgemäßen Hilfstoff mittels einer *in vitro* Waschmethode bestimmt.

Als Beleg der verbesserten Auswaschbarkeit und verminderten Fleckenbildung der erfindungsgemäßen Zubereitungen wurden in vitro Untersuchungen durchgeführt, deren Ergebnisse in Tabelle 1 dargestellt sind.

Es wurden verschiedene Sonnenschutzemulsionen hinsichtlich der Bildung von gelben Flecken über einen in vitro Auftragungs-/ Waschzyklus untersucht. Es wurden dabei weiße vorgewaschene Baumwollmonitore (100% Baumwolle) verwendet. Dazu wurden je 50 mg der Test-Formulierung gleichmäßig auf PMMA Schönberg Platten (5,0 x 5,0 cm) verteilt und direkt mittels Andruck auf das Testtextil übertragen. Im Anschluss wurden die verfleckten Baumwollproben für 12h unter Laborbedingungen an der Luft getrocknet.

Nach der Trocknung erfolgte eine farbmetrische Charakterisierung der entstandenen Initial-Verfleckung durch Messung des Gelbgrades mit dem Farbmessgerät spectro-color (Dr. Lange); Farbmess-Software: spectral-QC, Version Messgeometrie: d/8°, Glanzkomponente ausgeschlossen, Lichtart: D65 (entsprechend mittlerem Tageslicht), Kalibrierstandard: LZM 268, Messöffnung: 10mm, Probenhintergrund: Unterlagepapier ohne optischen Aufheller, Prüfklima: 21°C (±1°C), 41% (±4%) rel. Luftfeuchte.

Zur Auswertung wurde die Veränderung des b-Wertes aus dem CIE-Lab Farbmesssystem herangezogen. Die B-Achse charakterisiert im CIE-Lab System den Farbeindruck Gelb/ Blau, wobei positive b-Werte für eine Zunahme des Gelbanteils stehen. Je höher der b-Wert desto größer ist der Gelbeindruck.

Nach dem Messvorgang erfolgte eine separate Wäsche der Testlappen im Färbe- und Waschechtheitsgerät Linitest Plus (Atlas) (60°C, 1h, 20rpm, Ariel Compact Pulverwaschmittel, 10 Metallkugeln als Beiladung) und im Anschluss ein Spülvorgang (20°C, 15min, Leitungswasser).

Nach Trocknung für 12h unter Laborbedingungen erfolgte erneut eine farbmetrische Charakterisierung der entstandenen Verfleckung durch Messung der Farbwerte wie bereits beschrieben mit dem Farbmessgerät spectro-color (Dr. Lange).

Die CIE-Lab System oder L*a*b*-Farbraum ist ein dreidimensionaler Messraum, in dem alle wahrnehmbaren Farben enthalten sind. Der Farbraum ist auf Grundlage der Gegenfarbentheorie konstruiert. Eine der wichtigsten Eigenschaften des L*a*b*-Farbmodells ist seine Geräteunabhängigkeit, das heißt, die Farben werden unabhängig von der Art ihrer Erzeugung und Wiedergabetechnik definiert.

Die entsprechende EU-Richtlinie ist DIN EN ISO 11664-4 "Farbmetrik- Teil 4: CIE 1976 L*a*b* Farbenraum". Die Koordinaten der CIELAB-Ebene werden gebildet aus dem Rot/ Grün-Wert a und dem Gelb/ Blau-Wert b. Die Helligkeitsachse L steht senkrecht auf dieser Ebene. Nach DIN 6174 sind L, a und b mit * zu schreiben, um sich gegen andere, z.B. das "Hunter-Lab"-System abzugrenzen.

**Tabelle 1: getestete Zubereitungen und deren Gelbwert-Reduktion von Flecken; db-Wert [%]**

| **INCI** | **Beispiel [%]** | | |
|---|---|---|---|
| | Rezeptur 1 | Rezeptur 2 | Rezeptur 3 |
| Piroctone | | | 0,50 |
| Piroctone Olamine | | 0,50 | |
| Butyl Methoxydibenzoylmethane | 5,00 | 5,00 | 5,00 |
| Myristyl Myristate | 1,00 | 1,00 | 1,00 |
| C18-38 Alkyl Hydroxystearoyl Stearate | 0,50 | 0,50 | 0,50 |
| Glyceryl Stearate | 1,00 | 1,00 | 1,00 |
| Sodium Cetearyl Sulfate | 0,15 | 0,15 | 0,15 |
| Silica Dimethyl Silylate | 0,50 | 0,50 | 0,50 |
| VP/Hexadecene Copolymer | 0,50 | 0,50 | 0,50 |
| Glycerin | 8,00 | 8,00 | 8,00 |
| Phenoxyethanol | 0,50 | 0,50 | 0,50 |
| Ethylparaben | 0,10 | 0,10 | 0,10 |
| Methylparaben | 0,30 | 0,30 | 0,30 |
| Cetearyl Alcohol | 1,00 | 1,00 | 1,00 |
| Acrylates/C 10-30 Alkyl Acrylate Crosspolymer | 0,05 | 0,05 | 0,05 |
| Xanthan Gum | 0,40 | 0,40 | 0,40 |
| Alcohol Denat. | 4,00 | 4,00 | 4,00 |
| Trisodium EDTA | 0,20 | 0,20 | 0,20 |
| Homosalate | 9,50 | 9,50 | 9,50 |
| Ethylhexyl Salicylate | 4,75 | 4,75 | 4,75 |
| Bis-Ethylhexyloxyphenol Methoxyphenyl Triazine | 3,50 | 3,50 | 3,50 |
| Titanium Dioxide | 3,50 | 3,50 | 3,50 |
| Phenylbenzimidazole Sulfonic Acid | 1,00 | 1,00 | 1,00 |
| Octocrylene | 9,50 | 9,50 | 9,50 |
| Parfum | q.s. | q.s. | q.s. |
| Sodium Hydroxide | q.s. | q.s. | q.s. |
| Aqua | ad 100 | ad 100 | ad 100 |
| | | | |
| Gelbwert-Reduktion db [%] | - | -35 | -28 |

**Fazit:** Die Auswaschbarkeit der durch Butyl Methoxydibenzoylmethane hervorgerufenen Textilverfärbung wird durch den Zusatz von Piroctone bzw. Piroctone Olamine im Sonnenschutzmittel verbessert.

### Beispiele

Die nachfolgenden Beispiele (Tabelle 2) sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

**Tabelle 2: Beispielzubereitungen**

| **INCI** | **Beispiel [%]** | | | | | | | |
|---|---|---|---|---|---|---|---|---|
| | Bsp. 1 | Bsp. 2 | Bsp. 3 | Bsp. 4 | Bsp. 5 | Bsp. 6 | Bsp. 7 | Bsp. 8 |
| Piroctone | 0,45 | 0,50 | 0,50 | 0,45 | | | | 0,45 |
| Piroctone Olamine | | | | | 0,45 | 0,10 | 0,45 | |
| Dibutyl Adipate | 3,00 | | | | | 3,00 | | |
| Butylene Glycol Dicaprylate/Dicaprate | 3,00 | | | | | 4,00 | 2,00 | 2,00 |
| C12-15 Alkyl Benzoate | 9,50 | | 2,00 | | | 5,00 | | |
| Myristyl Myristate | | 1,00 | 1,00 | 1,00 | 1,00 | | | |
| C18-38 Alkyl Hydroxystearoyl Stearate | | 0,50 | 0,50 | 0,50 | 0,50 | 1,00 | | |
| C18-36 Acid Triglyceride | | | | | | | 1,00 | 1,00 |
| Glyceryl Stearate SE | | 1,00 | 1,00 | 1,00 | 1,00 | | | |
| Sodium Cetearyl Sulfate | | 0,15 | 0,15 | 0,15 | 0,15 | | | |
| Glyceryl Stearate | | | | | | 1,00 | | |
| Sodium Stearoyl Glutamate | | | | | | 0,30 | | |
| Ceteareth-20 | | | | | | | 1,50 | 1,50 |
| Silica Dimethyl Silylate | | 0,50 | 0,50 | 0,50 | 0,50 | 1,00 | | |
| Acrylates/Octylacrylamide Copolymer | 1,00 | | | | | | | |
| VP/Hexadecene Copolymer | | 0,50 | 0,50 | 0,50 | 0,50 | 0,50 | 1,00 | 1,00 |
| Glycerin | 2,50 | 8,00 | 8,00 | 8,00 | 8,00 | 7,50 | 5,00 | 5,00 |
| Phenoxyethanol | | 0,50 | 0,50 | 0,50 | 0,50 | 0,50 | 0,50 | 0,50 |
| Methylparaben | | 0,30 | 0,30 | 0,30 | 0,30 | | 0,30 | 0,30 |
| Ethylparaben | | | | | | | 0,20 | 0,20 |
| Cetearyl Alcohol | | 1,00 | 1,00 | 1,00 | 1,00 | | | |
| Acrylates/C10-30 Alkyl Acrylate Crosspolymer | | 0,10 | 0,10 | 0,10 | 0,10 | 0,15 | 0,40 | 0,40 |
| Xanthan Gum | | 0,40 | 0,40 | 0,40 | 0,40 | 0,40 | | |
| Stearyl Alcohol | | | | | | 1,00 | | |
| Alcohol Denat. | 50,00 | 4,00 | 4,00 | 4,00 | 4,00 | 3,00 | 4,00 | 4,00 |
| Trisodium EDTA | | 0,20 | 0,20 | 0,20 | 0,20 | 0,20 | 0,20 | 0,20 |
| Homosalate | 9,00 | 9,50 | 9,50 | 9,50 | 9,50 | | 9,00 | 9,00 |
| Octocrylene | 9,00 | 9,50 | 9,50 | 9,50 | 9,50 | | 8,00 | 8,00 |
| Ethylhexyl Salicylate | 4,50 | 4,75 | 4,75 | 4,75 | 4,75 | 4,75 | 4,75 | 4,75 |
| Bis-Ethylhexyloxyphenol Methoxyphenyl Triazine | 2,00 | 3,50 | 3,50 | 3,50 | 3,50 | 4,00 | 3,50 | 3,50 |
| Butyl Methoxydibenzoylmethane | 4,50 | 4,75 | 4,75 | 4,75 | 4,75 | 4,75 | 4,50 | 4,50 |
| Polysilicone-15 | 1,00 | | | | | | | |
| Phenylbenzimidazole Sulfonic Acid | | 1,00 | 1,00 | 1,00 | 1,00 | 1,00 | 1,50 | 1,50 |
| Ethylhexyl Triazone | | | | | | 3,00 | | |
| Perfume | q.s. | q.s. | q.s. | q.s. | q.s. | q.s. | q.s. | q.s. |
| Sodium Hydroxide | | q.s. | q.s. | q.s. | q.s. | q.s. | q.s. | q.s. |
| Aqua | ad 100 | ad 100 | ad 100 | ad 100 | ad 100 | ad 100 | ad 100 | ad 100 |

## Patentansprüche

1. Verfahren zur Erleichterung der Auswaschbarkeit von 4-(tert.-Butyl)-4'-methoxydibenzoylmethan enthaltenden kosmetischen Zubereitungen aus Textilien, **dadurch gekennzeichnet, dass** dem Kosmetikum 1-Hydroxy-4-methyl-6-(2,4,4-trimethylpentyl)-2(1H)-pyridon (Pirocton) und/oder dessen Monoethanolamin-Salz zugesetzt wird.

2. Verwendung von 1-Hydroxy-4-methyl-6-(2,4,4-trimethylpentyl)-2(1 H)-pyridon (Pirocton) und/oder dessen Monoethanolamin-Salz in 4-(tert.-Butyl)-4'-methoxydibenzoylmethan enthaltenden kosmetischen Zubereitungen zur Erleichterung der Auswaschbarkeit der UV-Lichtschutzfilter aus mit den Zubereitungen kontaminierten Textilien.

3. Verfahren nach Anspruch 1 oder Verwendung nach Anspruch 2, **dadurch gekennzeichnet, dass** die Zubereitung 4-(tert.-Butyl)-4'-methoxydibenzoylmethan in einer Menge von 0,1 bis 5 Gewichts-%, bezogen auf das Gesamtgewicht der Zubereitung, enthält.

4. Verfahren oder Verwendung nach einem der vorhergehenden Ansprüche, **dadurch gekennzeichnet, dass** die Zubereitung1-Hydroxy-4-methyl-6-(2,4,4-trimethylpentyl)-2(1H)-pyridon (Pirocton) in einer Gesamtmenge von 0,1 bis 0,5 Gewichts-%, bezogen auf das Gesamtgewicht der Zubereitung, enthält.

5. Verfahren oder Verwendung nach einem der vorhergehenden Ansprüche, **dadurch gekennzeichnet, dass** das 1-Hydroxy-4-methyl-6-(2,4,4-trimethylpentyl)-2(1 H)-pyridon (Pirocton) in Form seines Monoethanolamin-Salzes (Piroctone Olamine) eingesetzt wird.

6. Verfahren oder Verwendung nach einem der vorhergehenden Ansprüche, **dadurch gekennzeichnet, dass** das Gewichtsverhältnis von 4-(tert.-Butyl)-4'-methoxydibenzoylmethan zu 1-Hydroxy-4-methyl-6-(2,4,4-trimethylpentyl)-2(1 H)-pyridon (Pirocton) von 10:1 bis 100: 1 beträgt.

7. Verfahren oder Verwendung nach einem der vorhergehenden Ansprüche, **dadurch gekennzeichnet, dass** die Zubereitung einen oder mehrere Emulgatoren gewählt aus der Gruppe der Verbindungen Glycerylstearatcitrat, Cetearylalkohol, Natriumcetearylsulfat, Glycerylstearat, Cetearylsulfosuccinat, Natriumstearoylglutamat, Polyglyceryl-3-methylglucosedistearat, Sucrose Polystearate, Stearinsäure, Polyglyceryl-10 Stearat, Kaliumcetylphosphat, enthält.

8. Verfahren oder Verwendung nach einem der vorhergehenden Ansprüche, **dadurch gekennzeichnet, dass** die Zubereitung einen oder mehrere UV-Filterenthält , die gewählt werden aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 2-Ethylhexyl-2-cyano-3,3-diphenylacrylat; 4-(Dimethylamino)-benzoesäure(2-ethylhexyl)ester; 4-(Dimethylamino)benzoesäureamylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; 2-(4'-Diethylamino-2'-hydoxybenzoyl)-benzoesäurehexylester; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titandioxid; Zinkoxid.

9. Verfahren oder Verwendung nach einem der vorhergehenden Ansprüche, **dadurch gekennzeichnet, dass** die Zubereitung eine oder mehrere Verbindungen gewählt aus der Gruppe der Verbindungen Ethylhexylglycerin, Polyglyceryl-2 Caprate, Propylenglycol, Butylenglycol, 2-Methylpropan-1,3-diol, 1,2-Pentandiol, 1,2-Hexandiol, 1,2-Octandiol und/oder 1,2-Decandiol enthält.

10. Verfahren oder Verwendung nach einem der vorhergehenden Ansprüche, **dadurch gekennzeichnet, dass** die Zubereitung Phenoxyethanol und/oder Methylparaben enthält.

11. Verfahren oder Verwendung nach einem der vorhergehenden Ansprüche, **dadurch gekennzeichnet, dass** die Zubereitung einen oder mehrere Wirkstoffe gewählt aus der Gruppe der Verbindungen Magnolienextrakt, alpha-Liponsäure, Folsäure, Phytoen, D-Biotin, Coenzym Q10, alpha-Glucosylrutin, Carnitin, Carnosin, Polydocanol, natürliche und/oder synthetische Isoflavonoide, Flavonoide, Kreatin, Kreatinin, Taurin, β-Alanin, Tocopherylacetat, Panthenol, Magnolol, Honokiol, Harnstoff, Hyaluronsäure, Dihydroxyaceton, 8-Hexadecen-1,16-dicarbonsäure, Glycyrrhetinsäure, Glucosylglyceride und/oder Licochalcon A enthält.

12. Verfahren oder Verwendung nach einem der vorhergehenden Ansprüche, **dadurch gekennzeichnet, dass** die Zubereitung frei ist von Propyl- und Butylparaben, 3-lod-2-propinylbutylcarbamat, 3-(4-Methylbenzyliden)-campher und 2-Hydroxy-4-methoxybenzophenon (Oxybenzon).

13. Verfahren oder Verwendung nach einem der vorhergehenden Ansprüche, **dadurch gekennzeichnet, dass** die Zubereitung C12-15 Alkylbenzoat, Cyclomethicon, Octyldodecanol, Capryl/Caprinsäure Triglyceride, Butylenglykol Dicaprylat/Dicaprat, Phenethylbenzoat, Cocoglyceride, Di-n-butyladipat und/oder Diisopropyladipat enthält.

14. Verfahren oder Verwendung nach einem der vorhergehenden Ansprüche, **dadurch gekennzeichnet, dass** die Zubereitung einen oder mehrere Komplexbildner enthält, gewählt aus der Gruppe der Verbindungen der EDTA, Aminopolycarboxylate, Polyphosphonate, Polyphosphate und komplexbildenden Säuren.

15. Verfahren oder Verwendung nach einem der vorhergehenden Ansprüche, **dadurch gekennzeichnet, dass** die Zubereitung eine oder mehrere Verbindungen aus der Gruppe der Polysaccharide enthält.

## Claims

1. Method for facilitating the ability to wash out cosmetic preparations comprising 4-(tert-butyl)-4'-methoxydibenzoylmethane from textiles, **characterized in that** 1-hydroxy-4-methyl-6-(2,4,4-trimethylpentyl)-2(1 H)-pyridone (piroctone) and/or the monoethanolamine salt thereof is added to the cosmetic.

2. Use of 1-hydroxy-4-methyl-6-(2,4,4-trimethylpentyl)-2(1H)-pyridone (piroctone) and/or the monoethanolamine salt thereof in cosmetic preparations comprising 4-(tert-butyl)-4'-methoxydibenzoylmethane for facilitating the ability of UV photoprotective filters to be washed out from textiles contaminated with the preparations.

3. Method according to Claim 1 or use according to Claim 2, **characterized in that** the preparation comprises 4-(tert-butyl)-4'-methoxydibenzoylmethane in an amount of 0.1 to 5% by weight, based on the total weight of the preparation.

4. Method or use according to any of the preceding claims, **characterized in that** the preparation comprises 1-hydroxy-4-methyl-6-(2,4,4-trimethylpentyl)-2(1H)-pyridone (piroctone) in a total amount of 0.1 to 0.5% by weight, based on the total weight of the preparation.

5. Method or use according to any of the preceding claims, **characterized in that** the 1-hydroxy-4-methyl-6-(2,4,4-trimethylpentyl)-2(1H)-pyridone (piroctone) is used in the form of its monoethanolamine salt (piroctone olamine).

6. Method or use according to any of the preceding claims, **characterized in that** the ratio by weight of 4-(tert-butyl)-4'-methoxydibenzoylmethane to 1-hydroxy-4-methyl-6-(2,4,4-trimethylpentyl)-2(1H)-pyridone (piroctone) is from 10:1 to 100:1.

7. Method or use according to any of the preceding claims, **characterized in that** the preparation comprises one or more emulsifiers selected from the group of compounds comprising glyceryl stearate citrate, cetearyl alcohol, sodium cetearyl sulfate, glyceryl stearate, cetearyl sulfosuccinate, sodium stearoyl glutamate, polyglyceryl-3 methylglucose distearate, sucrose polystearates, stearic acid, polyglyceryl-10 stearate, potassium cetyl phosphate.

8. Method or use according to any of the preceding claims, **characterized in that** the preparation comprises one or more UV filters selected from the group of compounds comprising 2-phenylbenzimidazole-5-sulfonic acid and/or salts thereof; phenylene-1,4-bis(2-benzimidazyl)-3,3'-5,5'-tetrasulfonic acid salts; 1,4-di(2-oxo-10-sulfo-3-bornylidenemethyl)benzene and salts thereof; 4-(2-oxo-3-bornylidenemethyl)benzenesulfonic acid salts; 2-methyl-5-(2-oxo-3-bornylidenemethyl)sulfonic acid salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methylbenzylidene)camphor; 3-benzylidenecamphor; ethylhexyl salicylate; terephthalidenedicamphorsulfonic acid; 2-ethylhexyl 2-cyano-3,3-diphenylacrylate; 2-ethylhexyl 4-(dimethylamino)benzoate; amyl 4-(dimethylamino)benzoate; di(2-ethylhexyl) 4-methoxybenzalmalonate; 2-ethylhexyl 4-methoxycinnamate; isoamyl 4-methoxycinnamate; 2-hydroxy-4-methoxybenzophenone, 2-hydroxy-4-methoxy-4'-methylbenzophenone; 2,2'-dihydroxy-4-methoxybenzophenone; homomenthyl salicylate; 2-ethylhexyl 2-hydroxybenzoate; dimethicodiethylbenzalmalonate; 3-(4-(2,2-bis ethoxycarbonylvinyl)phenoxy)propenyl)methoxysiloxane / dimethylsiloxane - copolymer; hexyl 2-(4'-diethylamino-2'-hydroxybenzoyl)benzoate; dioctylbutylamidotriazone (INCI: Diethylhexyl Butamidotriazone); 2,4-bis[5-1(dimethylpropyl)benzoxazol-2-yl-(4-phenyl)imino]-6-(2-ethylhexyl)imino-1,3,5-triazine with (CAS No. 288254-16-0); 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine); tris(2-ethylhexyl) 4,4',4"-(1,3,5-triazine-2,4,6-triyltriimino)trisbenzoate (also: 2,4,6-tris[anilino-(p-carbo-2'-ethyl-1'-hexyloxy)]-1,3,5-triazine (INCI: Ethylhexyl Triazone); 2,4,6-tribiphenyl-4-yl-1,3,5-triazine; merocyanine; titanium dioxide; zinc oxide.

9. Method or use according to any of the preceding claims, **characterized in that** the preparation comprises one or more compounds selected from the group of compounds comprising ethylhexylglycerin, polyglyceryl-2 caprate, propylene glycol, butylene glycol, 2-methylpropane-1,3-diol, 1,2-pentanediol, 1,2-hexanediol, 1,2-octanediol and/or 1,2-decanediol.

10. Method or use according to any of the preceding claims, **characterized in that** the preparation comprises phenoxyethanol and/or methylparaben.

11. Method or use according to any of the preceding claims, **characterized in that** the preparation comprises one or more active ingredients selected from the group of compounds comprising magnolia extract, alpha-lipoic acid, folic acid, phytoene, D-biotin, coenzyme Q10, alpha-glucosylrutin, carnitine, carnosine, polidocanol, natural and/or synthetic isoflavonoids, creatine, creatinine, taurine, β-alanine, tocopheryl acetate, panthenol, magnolol, honokiol, urea, hyaluronic acid, dihydroxyacetone, 8-hexadecene-1,16-dicarboxylic acid, glycyrrhetic acid, glucosyl glycerides and/or licochalcone A.

12. Method or use according to any of the preceding claims, **characterized in that** the preparation is free of propylparaben and butylparaben, 3-iodo-2-propynyl butylcarbamate, 3-(4-methylbenzylidene)camphor and 2-hydroxy-4-methoxybenzophenone (oxybenzone).

13. Method or use according to any of the preceding claims, **characterized in that** the preparation comprises C12-15 alkyl benzoate, cyclomethicone, octyldodecanol, caprylic/capric acid triglycerides, butylene glycol dicaprylate/dicaprate, phenethyl benzoate, cocoglycerides, di-n-butyl adipate and/or diisopropyl adipate.

14. Method or use according to any of the preceding claims, **characterized in that** the preparation comprises one or more chelating agents selected from the group of compounds comprising EDTA, aminopolycarboxylates, polyphosphonates, polyphosphates and chelating acids.

15. Method or use according to any of the preceding claims, **characterized in that** the preparation comprises one or more compounds from the group of polysaccharides.

## Revendications

1. Procédé visant à faciliter la rinçabilité de préparations cosmétiques contenant du 4-(tert.-butyl)-4'-méthoxydibenzoylméthane à partir de textiles, **caractérisé en ce que** de la 1-hydroxy-4-méthyl-6-(2,4,4-triméthylpentyl)-2(1H)-pyridone (piroctone) et/ou son sel de monoéthanolamine sont ajoutés au cosmétique.

2. Utilisation de 1-hydroxy-4-méthyl-6-(2,4,4-triméthylpentyl)-2(1H)-pyridone (piroctone) et/ou son sel de monoéthanolamine dans des préparations cosmétiques contenant du 4-(tert.-butyl)-4'-méthoxydibenzoylméthane pour faciliter la rinçabilité des filtres de protection contre la lumière UV à partir de textiles contaminés avec les préparations.

3. Procédé selon la revendication 1 ou utilisation selon la revendication 2, caractérisé ou **caractérisée en ce que** la préparation contient du 4-(tert.-butyl)-4'-méthoxydibenzoylméthane en une quantité de 0,1 à 5 % en poids, par rapport au poids total de la préparation.

4. Procédé ou utilisation selon l'une quelconque des revendications précédentes, caractérisé ou **caractérisée en ce que** la préparation contient de la 1-hydroxy-4-méthyl-6-(2,4,4-triméthylpentyl)-2(1H)-pyridone (piroctone) en une quantité totale de 0,1 à 0,5 % en poids, par rapport au poids total de la préparation.

5. Procédé ou utilisation selon l'une quelconque des revendications précédentes, caractérisé ou **caractérisée en ce que** la 1-hydroxy-4-méthyl-6-(2,4,4-triméthylpentyl)-2(1H)-pyridone (piroctone) est utilisée sous la forme de son sel de monoéthanolamine (piroctone olamine).

6. Procédé ou utilisation selon l'une quelconque des revendications précédentes, caractérisé ou **caractérisée en ce que** le rapport en poids entre le 4-(tert.-butyl)-4'-méthoxydibenzoylméthane et la 1-hydroxy-4-méthyl-6-(2,4,4-triméthylpentyl)-2(1H)-pyridone (piroctone) est de 10:1 à 100:1.

7. Procédé ou utilisation selon l'une quelconque des revendications précédentes, caractérisé ou **caractérisée en ce que** la préparation contient un ou plusieurs émulsifiants choisis dans le groupe de composés constitué par le stéarate-citrate de glycéryle, l'alcool cétéarylique, le sulfate de cétéaryle sodique, le stéarate de glycéryle, le sulfosuccinate de cétéaryle, le glutamate de stéaroyle sodique, le distéarate de polyglycéryl-3-méthylglucose, le polystéarate de sucrose, l'acide stéarique, le stéarate de polyglycéryle-10, le phosphate de cétyle potassique.

8. Procédé ou utilisation selon l'une quelconque des revendications précédentes, caractérisé ou **caractérisée en ce que** la préparation contient un ou plusieurs filtres UV, qui sont choisis dans le groupe de composés constitué par l'acide 2-phénylbenzimidazole-5-sulfonique et/ou ses sels, les sels de l'acide phénylène-1,4-bis-(2-benzimidazyl)-3,3'-5,5'-tétrasulfonique, le 1,4-di(2-oxo-10-sulfo-3-bornylidène-méthyl)-benzène et ses sels, les sels de l'acide 4-(2-oxo-3-bornylidène-méthyl)benzène-sulfonique, les sels de l'acide 2-méthyl-5-(2-oxo-3-bornylidène-méthyl)sulfonique, le 2,2'-méthylène-bis-(6-(2H-benzotriazol-2-yl)-4-(1,1,3,3-tétraméthylbutyl)-phénol), le 2-(2H-benzotriazol-2-yl)-4-méthyl-6-[2-méthyl-3-[1,3,3,3-tétraméthyl-1-[(triméthylsilyl)oxy]disiloxanyl]propyl]-phénol, le 3-(4-méthylbenzylidène)camphre, le 3-benzylidène-camphre, le salicylate d'éthylhexyle, l'acide téréphtalidène-dicamphre-sulfonique, l'acrylate de 2-éthylhexyl-2-cyano-3,3-diphényle, l'ester 2-éthylhexylique de l'acide 4-(diméthylamino)-benzoïque, l'ester amylique de l'acide 4-(diméthylamino)benzoïque, l'ester di(2-éthylhexylique) de l'acide 4-méthoxybenzalmalonique, l'ester 2-éthylhexylique de l'acide 4-méthoxycinnamique, l'ester isoamylique de l'acide 4-méthoxycinnamique, la 2-hydroxy-4-méthoxybenzophénone, la 2-hydroxy-4-méthoxy-4'-méthylbenzophénone, la 2,2'-dihydroxy-4-méthoxybenzophénone, le salicylate d'homomenthyle, le 2-hydroxybenzoate de 2-éthylhexyle, le benzalmalonate de diméthicodiéthyle, le copolymère de 3-(4-(2,2-bis-éthoxycarbonylvinyl)-phénoxy)propényl)-méthoxysiloxane/diméthylsiloxane, l'ester hexylique de l'acide 2-(4'-diéthylamino-2'-hydroxybenzoyl)-benzoïque, la dioctylbutylamidotriazone (INCI : Diethylhexyl-Butamidotriazone), la 2,4-bis-[5-1-(diméthylpropyl)benzoxazol-2-yl-(4-phényl)-imino]-6-(2-éthylhexyl)-imino-1,3,5-triazine de n° CAS 288254-16-0), la 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazin) ; l'ester tris-(2-éthylhexylique) de l'acide 4,4',4"-(1,3,5-triazine-2,4,6-triyltriimino)-tris-benzoïque (également : 2,4,6-tris-[anilino-(p-carbo-2'-éthyl-1'-hexyloxy)]-1,3,5-triazine (INCI : Ethylhexyl Triazone), la 2,4,6-tribiphényl-4-yl-1,3,5-triazine, la mérocyanine, le dioxyde de titane, l'oxyde de zinc.

9. Procédé ou utilisation selon l'une quelconque des revendications précédentes, caractérisé ou **caractérisée en ce que** la préparation contient un ou plusieurs composés choisis dans le groupe de composés constitué par l'éthylhexylglycérine, le caprate de polyglycéryle-2, le propylène glycol, le butylène glycol, le 2-méthylpropane-1,3-diol, le 1,2-pentanediol, le 1,2-hexanediol, le 1,2-octanediol et/ou le 1,2-décanediol.

10. Procédé ou utilisation selon l'une quelconque des revendications précédentes, caractérisé ou **caractérisée en ce que** la préparation contient du phénoxyéthanol et/ou du méthylparabène.

11. Procédé ou utilisation selon l'une quelconque des revendications précédentes, caractérisé ou **caractérisée en ce que** la préparation contient un ou plusieurs agents actifs choisis dans le groupe de composés constitué par l'extrait de magnolia, l'acide alpha-liponique, l'acide folique, le phytoène, la D-biotine, la coenzyme Q10, l'alpha-glucosylrutine, la carnitine, la carnosine, le polydocanol, les isoflavonoïdes naturels et/ou synthétiques, les flavonoïdes, la créatine, la créatinine, la taurine, la β-alanine, l'acétate de tocophéryle, le panthénol, le magnolol, l'honokiol, l'urée, l'acide hyaluronique, la dihydroxyacétone, l'acide 8-hexadécène-1,16-dicarboxylique, l'acide glycyrrhétinique, le glucosylglycéride et/ou la licochalcone A.

12. Procédé ou utilisation selon l'une quelconque des revendications précédentes, caractérisé ou **caractérisée en ce que** la préparation est exempte de propyl- et butylparabène, de carbamate de 3-iodo-2-propinylbutyle, de 3-(4-méthylbenzylidène)-camphre et de 2-hydroxy-4-méthoxybenzophénone (oxybenzone) .

13. Procédé ou utilisation selon l'une quelconque des revendications précédentes, caractérisé ou **caractérisée en ce que** la préparation contient un benzoate d'alkyle en C12-15, de la cyclométhicone, de l'octyldodécanol, des triglycérides d'acide caprylique/caprique, du dicaprylate/dicaprate de butylène glycol, du benzoate de phénéthyle, des glycérides de coco, de l'adipate de di-n-butyle et/ou de l'adipate de diisopropyle.

14. Procédé ou utilisation selon l'une quelconque des revendications précédentes, caractérisé ou **caractérisée en ce que** la préparation contient un ou plusieurs complexants, choisis dans le groupe de composés constitué par l'EDTA, les aminopolycarboxylates, les polyphosphonates, les polyphosphates et les acides complexants.

15. Procédé ou utilisation selon l'une quelconque des revendications précédentes, caractérisé ou **caractérisée en ce que** la préparation contient un ou plusieurs composés du groupe des polysaccharides.
